(19) Europäisches Patentamt
European Patent Office
Office européen des brevets

(11) EP 2 076 547 B1

(12) **EUROPÄISCHE PATENTSCHRIFT**

(45) Veröffentlichungstag und Bekanntmachung des Hinweises auf die Patenterteilung:
**09.03.2011 Patentblatt 2011/10**

(21) Anmeldenummer: **07820798.2**

(22) Anmeldetag: **02.10.2007**

(51) Int Cl.:
***C08F 2/00*** (2006.01) ***C08F 2/10*** (2006.01)
***C08F 2/16*** (2006.01) ***C08F 2/18*** (2006.01)
***A61L 15/60*** (2006.01)

(86) Internationale Anmeldenummer:
**PCT/EP2007/060416**

(87) Internationale Veröffentlichungsnummer:
**WO 2008/040714 (10.04.2008 Gazette 2008/15)**

(54) **VERFAHREN ZUR HERSTELLUNG WASSERABSORBIERENDER POLYMERPARTIKEL DURCH POLYMERISATION VON TROPFEN EINER MONOMERLÖSUNG**

METHOD FOR THE PRODUCTION OF WATER ABSORBENT POLYMER PARTICLES BY POLYMERIZING DROPS OF A MONOMER SOLUTION

PROCÉDÉ DE PRÉPARATION DE PARTICULES DE POLYMÈRE ABSORBANT L'EAU GRÂCE À UNE POLYMÉRISATION DE GOUTTES D'UNE SOLUTION DE MONOMÈRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **05.10.2006 EP 06121838**

(43) Veröffentlichungstag der Anmeldung:
**08.07.2009 Patentblatt 2009/28**

(73) Patentinhaber: **BASF SE**
**67056 Ludwigshafen (DE)**

(72) Erfinder:
- **LÖSCH, Dennis**
  **67122 Altrip (DE)**
- **WEISMANTEL, Matthias**
  **63637 Jossgrund (DE)**
- **KRÜGER, Marco**
  **68219 Mannheim (DE)**
- **ZIEMER, Antje**
  **68199 Mannheim (DE)**

(56) Entgegenhaltungen:
**EP-A- 1 424 346 WO-A-2006/042704**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 076 547 B1

## Beschreibung

Die vorliegende Erfindung betrifft ein Verfahren zur Herstellung wasserabsorbierender Polymerpartikel durch Polymerisation von Tropfen, enthaltend mindestens ein Monomer, in einer die Tropfen umgebenden Gasphase, wobei die Tropfen erzeugt werden, indem eine erste Monomerlösung mit einer zweiten Monomerlösung umschlossen wird und die zweite Monomerlösung zu einem höher vernetzten Polymer polymerisiert als die erste Monomerlösung.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP 348 180 A1, JP 05/132503 A, WO 96/40427 A1, US 5,269,980, DE 103 14 466 A1, DE 103 40 253 A1 und DE 10 2004 024 437 A1, WO 2006/077054 A1 sowie der älteren deutschen Anmeldung mit dem Aktenzeichen 102006001596.7 und der älteren PCT-Anmeldung mit dem Aktenzeichen PCT/EP2006/062252 beschrieben.

JP 05/132503 A offenbart ein Sprühpolymerisationsverfahren durch Redox-Polymerisation, wobei die Komponenten des Redox-Initiators erst hinter der Düse vermischt werden.

WO 2006/077054 A1 beschreibt ein Verfahren, bei dem durch Verwendung tensidischer Vernetzer ein Vernetzergradient erzeugt wird.

Die ältere PCT-Anmeldung mit dem Aktenzeichen PCT/EP2006/062252 beschreibt ein Verfahren, wobei mittels einer Zweistoffdüse ein Konzentrationsgradient in den Tropfen erzeugt wird.

DE 10 2004 042 946 A1, DE 10 2004 042 948 A1 und DE 10 2004 042 955 A1 sowie die ältere deutsche Anmeldung mit dem Aktenzeichen 102005019398.6 beschreiben die Herstellung von Verdickern durch Sprühpolymerisation.

Aufgabe der vorliegenden Erfindung war die Bereitstellung eines verbesserten Verfahrens zur Herstellung wasserabsorbierender Polymerpartikel durch Polymerisation von Tropfen einer Monomerlösung in einer der Tropfen umgebenden Gasphase.

Gelöst wurde die Aufgabe durch ein Verfahren zur Herstellung wasserabsorbierender Polymerpartikel durch Polymerisation von Tropfen, enthaltend

a) mindestens ein ethylenisch ungesättigtes Monomer,
b) mindestens einen Vernetzer,
c) mindestens einen Initiator,
d) Wasser,

in einer die Tropfen umgebenden Gasphase, wobei die Tropfen erzeugt werden, indem eine erste Monomerlösung von einer zweiten Monomerlösung umhüllt wird, dadurch gekennzeichnet, dass die zweite Monomerlösung zu einem höher vernetzten Polymer polymerisiert als die erste Monomerlösung.

Dadurch, dass die zweite Monomerlösung zu einem höher vernetzten Polymer polymerisiert, werden in einem Schritt wasserabsorbierende Polymerpartikel mit einem Vernetzungsgradienten erhalten. Die höhere Vernetzung kann beispielsweise durch eine höhere Vernetzerkonzentration und/oder einen wirksameren Vernetzer in der zweiten Monomerlösung erreicht werden.

Die molare Vernetzerkonzentration in der zweiten Monomerlösung ist typischerweise mindestens 10%, vorzugsweise mindestens 20%, bevorzugt mindestens 50%, besonders bevorzugt mindestens 100%, ganz besonders bevorzugt mindestens 200%, höher ist als in der ersten Monomerlösung

Die erste Monomerlösung enthält vorzugsweise mindestens 0,2 Gew.-%, bevorzugt mindestens 0,4 Gew.-%, besonders bevorzugt mindestens 0,6 Gew.-%, ganz besonders bevorzugt mindestens 0,8 Gew.-%, Vernetzer b), jeweils bezogen auf Monomer a).

Die zweite Monomerlösung enthält vorzugsweise mindestens 0,6 Gew.-%, bevorzugt mindestens 0,8 Gew.-%, besonders bevorzugt mindestens 1,5 Gew.-%, ganz besonders bevorzugt mindestens 3,0 Gew.-%, Vernetzer b), jeweils bezogen auf Monomer a).

In einer bevorzugten Ausführungsform der vorliegenden Erfindung wird die zweite Monomerlösung über einen die Zuführung der ersten Monomerlösung umschließenden Ringspalt dosiert. Der Ringspalt hat eine Spaltbreite von vorzugsweise 25 µm bis 250 µm, besonders bevorzugt von 50 bis 200 µm, ganz besonders bevorzugt von 100 bis 150 µm.

Die erzeugten Tropfen weisen einen mittleren Durchmesser von vorzugsweise mindestens 200 µm, besonders

bevorzugt von mindestens 250 μm, ganz besonders bevorzugt von mindestens 300 μm, auf, wobei der Tropfendurchmesser durch Lichtstreuung bestimmt werden kann.

Die Tropfen sind vorzugsweise monodispers, besonders bevorzugt weisen weniger als 10 Gew.-% der Tropfen einen Durchmesser auf, der mehr als 50% vom mittleren Durchmesser abweicht.

Die gemäß dem erfindungsgemäßen Verfahren erhältlichen wasserabsorbierenden Polymerpartikel weisen eine Permeabilität (SFC) von typischerweise mindestens 10 x $10^{-7}$ $cm^3s/g$, vorzugsweise mindestens 30 x $10^{-7}$ *$cm^3s/g$,* bevorzugt mindestens 50 x $10^{-7}$ $cm^3s/g$, besonders bevorzugt mindestens 70 x $10^{-7}$ $cm^3s/g$, ganz besonders bevorzugt mindestens 90 x $10^{-7}$ $cm^3s/g$, auf. Die Permeabilität (SFC) der wasserabsorbierenden Polymerpartikel beträgt üblicherweise weniger als 250 x $10^{-7}$ $cm^3s/g$.

Die gemäß dem erfindungsgemäßen Verfahren erhältlichen wasserabsorbierenden Polymerpartikel weisen eine Absorption unter einem Druck von 4,83 kPa (AUL0.7psi) von typischerweise mindestens 15 g/g, vorzugsweise von mindestens 20 g/g, bevorzugt mindestens 25 g/g, besonders bevorzugt von mindestens 27, ganz besonders bevorzugt von mindestens 29 g/g, auf. Die Absorption unter einem Druck von 4,83 kPa (AUL0.7psi) der wasserabsorbierenden Polymerpartikel beträgt üblicherweise weniger als 50 g/g.

Die gemäß dem erfindungsgemäßen Verfahren erhältlichen wasserabsorbierenden Polymerpartikel weisen eine Zentrifugenretentionskapazität (CRC) von typischerweise mindestens 20 g/g, vorzugsweise mindestens 25 g/g, bevorzugt mindestens 30 g/g, besonders bevorzugt mindestens 32 g/g, ganz besonders bevorzugt mindestens 34 g/g, auf. Die Zentrifugenretentionskapazität (CRC) der wasserabsorbierenden Polymerpartikel beträgt üblicherweise weniger als 50 g/g.

Die gemäß dem erfindungsgemäßen Verfahren erhältlichen wasserabsorbierenden Polymerpartikel weisen einen Gehalt an Extrahierbaren von typischerweise weniger als 15 Gew.-%, vorzugsweise weniger als 10 Gew.-%, bevorzugt weniger als 5 Gew.-%, besonders bevorzugt weniger als 4 Gew.-%, ganz besonders bevorzugt weniger als 3 Gew.-%, auf.

Das erfindungsgemäße Verfahren ermöglicht die Herstellung von wasserabsorbierenden Polymerpartikeln mit sehr gleichmäßiger Vernetzungsdichte an der Partikeloberfläche.

Bei der Messung des E-Moduls der äußeren Partikeloberfläche weisen üblicherweise weniger als 50%, vorzugsweise weniger als 40%, bevorzugt weniger als 30%, besonders bevorzugt weniger als 25%, ganz besonders bevorzugt weniger als 20%, ein E-Modul von weniger als 60% des mittleren E-Moduls auf.

Die gemäß dem erfindungsgemäßen Verfahren erhältlichen wasserabsorbierenden Polymerpartikel weisen ein mittleres E-Modul der äußeren Partikeloberfläche von typischerweise mindestens 50 kPa, vorzugsweise mindestens 90 kPa, bevorzugt mindestens 120 kPa, besonders bevorzugt mindestens 150 kPa, ganz besonders bevorzugt mindestens 180 kPa, auf. Das mittlere E-Modul der äußeren Partikeloberfläche der wasserabsorbierenden Polymerpartikel beträgt üblicherweise weniger als 500 kPa.

Der mittlere Durchmesser der gemäß dem erfindungsgemäßen Verfahren erhältlichen wasserabsorbierenden Polymerpartikel beträgt vorzugsweise mindestens 200 μm, besonders bevorzugt von 250 bis 600 μm, ganz besonders von 300 bis 500 μm, wobei der Partikeldurchmesser durch Lichtstreuung bestimmt werden kann und den volumengemittelten mittleren Durchmesser bedeutet. 90% der Polymerpartikel weisen einen Durchmesser von vorzugsweise 100 bis 800 μm, besonders bevorzugt von 150 bis 700 μm, ganz besonders bevorzugt von 200 bis 600 μm, auf.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die bevorzugten Monomere a) haben mindestens eine Säuregruppe, wobei die Säuregruppen vorzugsweise zumindest teilweise neutralisiert sind.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder

Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

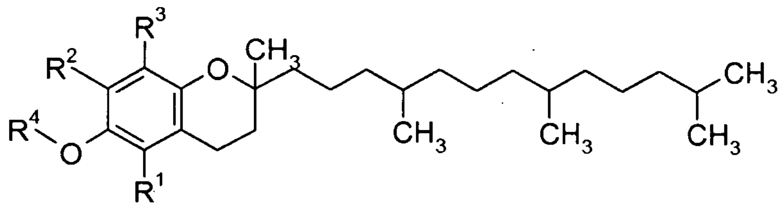

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth) acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins.

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in

jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 1 Gew.-%, bezogen auf die Monomeren a).

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Polymerisationsinhibitoren können auch durch Absorption, beispielsweise an Aktivkohle, entfernt werden.

Der Feststoffgehalt der Monomerlösung beträgt vorzugsweise mindestens 35 Gew.-%, bevorzugt mindestens 38 Gew.-%, besonders bevorzugt mindestens 40 Gew.-%, ganz besonders bevorzugt mindestens 42 Gew.-%. Dabei ist der Feststoffgehalt die Summe aller nach der Polymerisation nichtflüchtigen Bestandteile. Dies sind Monomer a), Vernetzer b) und Initiator c).

Die Monomerlösung wird zur Polymerisation in der Gasphase vertropft. Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d.h. Gase, die unter Reaktionsbedingungen nicht in die Polymerisation eingreifen, beispielsweise Stickstoff und/oder Wasserdampf.

Bei der Vertropfung wird eine Monomerlösung unter Ausbildung von Tropfen in die Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird. In einer bevorzugten Ausführungsform wird die Vertropferplatte nicht angeregt.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d.h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner 100. Der Druckverlust über die Bohrung beträgt vorzugsweise weniger als 2,5 bar, besonders bevorzugt weniger als 1,5 bar, ganz besonders bevorzugt weniger als 1 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks. Der Durchmesser der Bohrungen wird an die gewünschte Tropfengröße angepasst.

Die Vertropfung kann aber auch mittels pneumatischer Ziehdüsen, Rotation, Zerschneiden eines Strahls oder schnell ansteuerbarer Mikroventildüsen durchgeführt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen. Bevorzugte Vorrichtungen zur Rotationsvertropfung werden beispielsweise in DE 43 08 842 A1 beschrieben

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Bevorzugt strömt die Gasphase als Trägergas durch den Reaktionsraum. Dabei kann das Trägergas im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt

zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1 %.

Die Polymerisation wird vorzugsweise in einer laminaren Gasströmung durchgeführt. Eine laminare Gasströmung ist eine Gasströmung, bei der sich die einzelnen Schichten der Strömung nicht vermischen, sondern parallel bewegen. Ein Maß für die Strömungsverhältnisse ist die Reynolds-Zahl (Re). Unterhalb einer kritischen Reynolds-Zahl ($Re_{krit}$) von 2300 ist die Gasströmung laminar. Die Reynolds-Zahl der laminaren Gasströmung beträgt vorzugsweise weniger als 2000, besonders bevorzugt weniger als 1500, ganz besonders bevorzugt weniger als 1000. Der untere Grenzfall der laminaren Inertgasströmung ist eine ruhende Inertgasatmosphäre (Re = 0), d.h., es wird nicht kontinuierlich Inertgas eingespeist.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,01 bis 5 m/s, vorzugsweise 0,02 bis 4 m/s, besonders bevorzugt 0,05 bis 3 m/s, ganz besonders bevorzugt 0,1 bis 2 m/s.

Das Trägergas wird zweckmäßigerweise vor dem Reaktor auf die Reaktionstemperatur vorgewärmt.

Die Reaktionstemperatur beträgt bei der thermisch induzierten Polymerisation vorzugsweise 70 bis 250°C, besonders bevorzugt 100 bis 220°C, ganz besonders bevorzugt 120 bis 200°C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das der Reaktionsraum verlassende Trägergas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt kann dem Reaktor in üblicher Weise entnommen werden, vorzugsweise am Boden über eine Förderschnecke, und gegebenenfalls bis zur gewünschten Restfeuchte und zum gewünschten Restmonomerengehalt getrocknet werden.

Die Polymerpartikel können zur weiteren Verbesserung der Eigenschaften nachvernetzt werden.

Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyepoxide, wie in EP 83 022 A2, EP 543 303 A1 und EP 937 736 A2 beschrieben, di- oder polyfunktionelle Alkohole, wie in DE 33 14 019 A1, DE 35 23 617 A1 und EP 450 922 A2 beschrieben, oder β-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Nachvernetzer beschrieben.

Die Menge an Nachvernetzer beträgt vorzugsweise 0,01 bis 1 Gew.-%, besonders bevorzugt 0,05 bis 0,5 Gew.-%, ganz besonders bevorzugt 0,1 bis 0,2 Gew.-%, jeweils bezogen auf das Polymer.

Die Nachvernetzung wird üblicherweise so durchgeführt, dass eine Lösung des Nachvernetzers auf das Hydrogel oder die trockenen Polymerpartikel aufgesprüht wird. Im Anschluss an das Aufsprühen wird thermisch getrocknet, wobei die Nachvernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige-Mischer, Bepex-Mischer, Nauta-Mischer, Processall-Mischer und Schugi-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine

beheizbare Schnecke. Besonders vorteilhaft wird in einem Wirbelschichttrockner gemischt und getrocknet.

Bevorzugte Trocknungstemperaturen liegen im Bereich 170 bis 250°C, bevorzugt 180 bis 220°C, und besonders bevorzugt 190 bis 210°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten.

Das erfindungsgemäße Verfahren ermöglicht die Herstellung wasserabsorbierender Polymerpartikel mit einer hohen Zentrifugenretentionskapazität (CRC), einer hohen

Absorption unter einem Druck von 4,83 kPa (AUL0.7psi), einer hohen Permeabilität (SFC) und wenig Extrahierbaren.

Ein weiterer Gegenstand der vorliegenden Erfindung sind wasserabsorbierende Polymerpartikel, die nach dem erfindungsgemäßen Verfahren erhältlich sind.

Ein weiterer Gegenstand der vorliegenden Erfindung sind wasserabsorbierende Polymerpartikel, wobei die Partikel eine Zentrifugenretentionskapazität (CRC) von mindestens 30 g/g, eine Permeabilität (SFC) von mindestens 30 x $10^{-7}$ $cm^3s/g$ und weniger als 30% der gemessenen E-Module der äußeren Partikeloberfläche einen Wert von weniger als 60% des mittleren E-Modules aufweisen.

Die gemäß dem erfindungsgemäßen Verfahren erhältlichen wasserabsorbierenden Polymerpartikel haben üblicherweise die Form von Hohlkugeln. Ein weiterer Gegenstand der vorliegenden Erfindung sind daher wasserabsorbierende Polymerpartikel, enthaltend mindestens einen Hohlraum im Partikelinneren.

Das Verhältnis von maximalem Durchmesser des Hohlraumes zu maximalem Durchmesser des Polymerpartikels beträgt vorzugsweise mindestens 0,1, besonders bevorzugt mindesten 0,3, ganz besonders bevorzugt mindestens 0,4.

Der Quotient aus mittlerem E-Modul der äußeren Partikeloberfläche und mittlerem E-Modul der Innenwand des Hohlraumes beträgt vorzugsweise mindesten 2,5, besonders bevorzugt mindestens 2,8, ganz besonders bevorzugt mindestens 3.

Die erfindungsgemäßen wasserabsorbierenden Polymerpartikel sind annähernd rund, d.h. die Polymerpartikel weisen eine mittlere Sphärizität von typischerweise mindestens 0,84, vorzugsweise mindestens 0,86, besonders bevorzugt mindestens 0,88, ganz besonders bevorzugt mindestens 0,9, auf. Die Sphärizität (SPHT) ist definiert als

$$SPHT = \frac{4\pi A}{U^2},$$

wobei A die Querschnittsfläche und U der Querschnittsumfang der Polymerpartikel ist. Die mittlere Sphärizität ist die volumengemittelte Sphärizität.

Die mittlere Sphärizität kann beispielsweise mit dem Bildanalysesystem Camsizer® (Retsch Technolgy GmbH; DE) bestimmt werden:

Zur Messung wird das Produkt über einen Trichter aufgegeben und mit einer Dosierrinne zum Fallschacht gefördert. Während die Partikel an einer Leuchtwand vorbeifallen werden sie wahlweise von einer Kamera erfasst. Die aufgenommenen Bilder werden von der Software entsprechend der ausgewählten Parameter ausgewertet.

Zur Charakterisierung der Rundheit wird die im Programm mit Sphärizität gekennzeichnete Messgröße herangezogen. Angegeben sind die mittleren, mit dem Volumen gewichteten Sphärizitäten, wobei das Volumen der Partikel über den Äquivalentdurchmesser $XC_{min}$ ermittelt werden. Zur Bestimmung des Äquivalentdurchmessers $XC_{min}$ wird der jeweils längste Sehnendurchmesser für insgesamt 32 unterschiedliche Raumrichtungen gemessen. Der Äquivalentdurchmesser $XC_{min}$ ist der kürzeste dieser 32 Sehnendurchmesser. Der Äquivalentdurchmesser $XC_{min}$ entspricht der Maschenweite eines Siebes, das das Partikel gerade noch passieren kann. Zur Erfassung der Partikel wird die sogenannte CCD-Zoom Kamera (CAM-Z) eingesetzt. Zur Steuerung der Dosierrinne wird ein Flächenbelegungsanteil von 0,5% vorgegeben.

Polymerpartikel mit relativ niedriger Sphärizität werden durch umgekehrte Suspensionspolymerisation erhalten, wenn die Polymerpartikel während oder nach der Polymerisation agglomeriert werden.

Die durch übliche Lösungspolymerisation (Gelpolymerisation) hergestellten wasserabsorbierende Polymerpartikel werden nach der Trocknung gemahlen und klassiert wobei unregelmäßige Polymerpartikel erhalten werden. Die mittlere Sphärizität dieser Polymerpartikel beträgt zwischen ca. 0,72 und ca. 0,78.

Ein weiterer Gegenstand der vorliegenden Erfindung sind Verfahren zur Herstellung von Hygieneartikeln, insbesondere Windeln, umfassend die Verwendung gemäß obengenannten Verfahrens hergestellter wasserabsorbierender Polymerpartikel.

Ein weiterer Gegenstand der vorliegenden Erfindung ist die Verwendung erfindungsgemäßer wasserabsorbierender Polymerpartikel in Hygieneartikeln, zur Verdickung von Abfällen, insbesondere medizinischen Abfällen, oder als

wasserrückhaltendes Mittel in der Landwirtschaft

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

<u>FlüssigKeitsweiterleitung (SFC Saline Flow (Conductivity)</u>

Die Flüssigkeitsweiterleitung einer gequollenen Gelschicht unter Druckbelastung von 0,3 psi (2070 Pa) wird, wie in EP 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 640 330 A1. Der Durchfluss wird automatisch erfasst.

Die Flüssigkeitsweiterleitung (SFC) wird wie folgt berechnet:

$$\text{SFC [cm}^3\text{s/g]} = (\text{Fg}(t=0) \text{x} \text{L0})/(\text{dxAxWP}),$$

wobei Fg(t=0) der Durchfluss an NaCl-Lösung in g/s ist, der anhand einer linearen Regressionsanalyse der Daten Fg (t) der Durchflussbestimmungen durch Extrapolation gegen t=0 erhalten wird, LO die Dicke der Gelschicht in cm, d die Dichte der NaCl-Lösung in *g/cm$^3$*, A die Fläche der Gelschicht in cm$^2$ und WP der hydrostatische Druck über der Gelschicht in dyn/cm$^2$ darstellt.

<u>mittleres E-Modul</u>

Zur Bestimmung des mittleren E-Moduls (Young Modul E) werden die wasserabsorbierenden Polymerpartikel in überschüssiger 0,9 gew.-%iger Kochsalzlösung für 30 Minuten gequollen. Auf die zu untersuchende Partikeloberfläche wird eine Mikropipette aus Glas mit einem inneren Durchmesser D von 50 μm aufgesetzt. Anschließend wird in der Mikropipette ein Unterdruck erzeugt, so dass die zu untersuchende Oberfläche des wasserabsorbierenden Polymerpartikels in die Mikropipette eingesaugt wird. Die Länge L ist die maximale Länge, die die Partikeloberfläche dabei in die Mikropipette eingesaugt wird. Der Unterdruck wird dabei so gewählt, dass die eingesaugte Partikeloberfläche die Form eines Meniskusses aufweist und die Länge L zwischen 5 und 10 μm beträgt. Die Länge L und die in der Mikropipette gegenüber der umgebenden Lösung gemessene zugehörige Druckdifferenz Δp werden notiert. Zu kleine Längen L vermindern die Genauigkeit der Messung, bei zu großen Längen L wird der lineare Messbereich verlassen, d.h. die Länge L ist nicht mehr proportional zur Druckdifferenz Δp. Dass der lineare Bereich verlassen wird, kann auch daran erkannt werden, dass die eingesaugte Partikeloberfläche an der Innenwand der Mikropipette anliegt.

Der Durchmesser der gequollenen Polymerpartikel sollte mindestens 250 μm betragen. Bei zu niedrigen Partikeldurchmessern wird bereits durch die Krümmung des Polymerpartikels ein Meniskus in der Mikropipette vorgetäuscht. Zur Messung kleinerer wasserabsorbierender Polymerpartikel müssen daher Mikropipetten mit einem kleineren Innendurchmesser verwendet werden, wobei auch der Bereich für die Länge L anzupassen ist.

Das E-Modul wird gemäß

$$E = \frac{3}{4\pi} \cdot \Delta p \cdot \frac{D}{L}$$

berechnet.

Die Messung wird mindestens 20 mal wiederholt. Das arithmetrische Mittel der erhaltenen Werte ist das mittlere E-Modul.

Die Deformation der wasserabsorbierenden Polymerpartikel während der Messung kann mittels eines digitalen

Bildaufnahmesystems aufgezeichnet und rechnerisch ausgewertet werden.

Die Abbildung 1 zeigt eine beispielhafte Messanordnung auf einem Objekträger eines Mikroskops. Hierbei haben die Bezugszeichen die folgenden Bedeutungen:

1 zur Bildauswertung
2 zur Druckmessung
3 zur Druckerzeugung
4 Lösung
5 Polymerpartikel

Werden wasserabsorbierende Partikel untersucht, die die Form von Hohlkugeln haben, so kann auch das E-Modul der Innenwand des Hohlraumes gemessen werden. Dazu werden die gequollenen wasserabsorbierenden Polymerpartikel mittels eines Skalpells durchgeschnitten.

Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Absorption unter Druck (AUL0.7psi Absorbency Under Load)

Die Absorption unter Druck der wasserabsorbierenden Polymerpartikel wird gemäß der von der EDANA (European Disposables and Nonwovens Association) empfohlenen Testmethode Nr. 442.2-02 "Absorption under pressure" bestimmt, wobei ein Gewicht mit 49 g/$cm^2$ (0,7 psi) statt eines Gewichts mit 21 g/$cm^2$ (0,3 psi) verwendet wird.

Extrahierbare (Extractable)

Die Gehalt an Extrahierbaren (extrahierbare Anteile) der wasserabsorbierenden Polymerpartikel wird gemäß der von der EDANA (European Disposables and Nonwovens Association) empfohlenen Testmethode Nr. 470.2-02 "Extractable" bestimmt.

Die EDANA-Testmethoden sind beispielsweise erhältlich bei der European Disposables and Nonwovens Association, Avenue Eugène Plasky 157, B-1030 Brüssel, Belgien.

Beispiele:

Beispiel 1 (Vergleichsbeispiel)

14,3 kg Natriumacrylat (37,5 gew.-%ige Lösung in Wasser), 1,4 kg Acrylsäure und 350 g Wasser wurden mit 22 g 15-fach ethoxiliertem Trimethylolpropantriacrylat gemischt. Die Lösung wurde in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 30 Bohrungen à 200 μm auf. Der Initiator wurde kurz vor dem Vertropfer über einen statischen Mischer in die Monomerlösung dosiert. Als Initiator wurde eine 3 gew.-%ige Lösung von 2,2'-Azobis[2-(2-imidazolin-2-yl)propan]dihydrochlorid in Wasser verwendet. Die Dosiergeschwindigkeit der Initiatorlösung betrug 1,1 kg/h.

Die wasserabsorbierenden Polymerpartikel hatten folgende Eigenschaften:

| | |
|---|---|
| CRC | 34,5 g/g |
| AUL0.7psi | 16,2 g/g |
| Extrahierbare | 4,0 Gew.-% |
| SFC | 0,9 x $10^{-7}$ $cm^3$s/g |

Das mittlere E-Modul der äußeren Partikeloberfläche betrug 60 kPa, das mittlere E-Modul der Innenwand des Hohlraumes betrug 30 kPa und der Quotient der mittleren E-Module betrug 2.

Das mittlere E-Modul ist der Mittelwert aus 20 Einzelmessungen. Insgesamt 4 Einzelmessungen des E-Moduls der äußeren Partikeloberfläche ergaben einen Wert von weniger als 36 kPa.

Der mittlere Partikeldurchmesser betrug 350 μm.

Beispiel 2 (Vergleichsbeispiel)

Die wasserabsorbierenden Polymerpartikel aus Beispiel 1 wurden mit einer Lösung aus 0,08 Gew.-% Ethylenglykoldiglycidiylether, 1,75 Gew.-% Wasser und 1,17 Gew.-% Propylenglykol, jeweils bezogen auf die wasserabsorbierenden Polymerpartikel, besprüht und 30 Minuten im Umlufttrockenschrank bei 120°C getrocknet.
Die wasserabsorbierenden Polymerpartikel hatten folgende Eigenschaften:

| | |
|---|---|
| CRC | 34,8 g/g |
| AUL0.7psi | 27,2 g/g |
| Extrahierbare | 2,9 Gew.-% |
| SFC | 16 x $10^{-7}$ $cm^3s/g$ |

Das mittlere E-Modul der äußeren Partikeloberfläche betrug 150 kPa, das mittlere E-Modul der Innenwand des Hohlraumes betrug 40 kPa und der Quotient der mittleren E-Module betrug 3,75.
Das mittlere E-Modul ist der Mittelwert aus 20 Einzelmessungen. Insgesamt 10 Einzelmessungen des E-Moduls der äußeren Partikeloberfläche ergaben einen Wert von weniger als 90 kPa.
Der mittlere Partikeldurchmesser betrug 350 µm.

Beispiel 3

14,3 kg Natriumacrylat (37,5 gew.-%ige Lösung in Wasser), 1,4 kg Acrylsäure und 350 g Wasser wurden mit 22 g 15-fach ethoxiliertem Trimethylolpropantriacrylat gemischt (erste Monomerlösung). 14,3 kg Natriumacrylat (37,5 gew.-%ige Lösung in Wasser), 1,4 kg Acrylsäure und 350 g Wasser wurden mit 44 g 15-fach ethoxiliertem Trimethylolpropantriacrylat gemischt (zweite Monomerlösung). Die Lösungen wurden in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit der ersten Monomerlösung betrug 16 kg/h. Die Dosiergeschwindigkeit der zweiten Monomerlösung betrug 1,6 kg/h. Die Vertropferplatte hatte 30 Bohrungen à 200 µm, wobei jede Bohrung von einem Ringspalt umgeben war. Der Initiator wurde kurz vor dem Vertropfer über statische Mischer in die Monomerlösungen dosiert. Als Initiator wurde eine 3 gew.-%ige Lösung von 2,2'-Azobis[2-(2-imidazolin-2-yl)propan]dihydrochlorid in Wasser verwendet. Die Dosiergeschwindigkeit der Initiatorlösung in die erste Monomerlösung betrug 1,1 kg/h. Die Dosiergeschwindigkeit der Initiatorlösung in die zweite Monomerlösung betrug 0,1 kg/h.
Die wasserabsorbierenden Polymerpartikel hatten folgende Eigenschaften:

| | |
|---|---|
| CRC | 34,5 g/g |
| AUL0.7psi | 22,1 g/g |
| Extrahierbare | 3,5 Gew.-% |
| SFC | 30 x $10^{-7}$ $cm^3s/g$ |

Das mittlere E-Modul der äußeren Partikeloberfläche betrug 90 kPa, das mittlere E-Modul der Innenwand des Hohlraumes betrug 40 kPa und der Quotient der mittleren E-Module betrug 2,25.
Das mittlere E-Modul ist der Mittelwert aus 20 Einzelmessungen. Insgesamt 3 Einzelmessungen des E-Moduls der äußeren Partikeloberfläche ergaben einen Wert von weniger als 54 kPa.
Der mittlere Partikeldurchmesser betrug 360 µm.

Beispiel 4

Es wurde verfahren wie unter Beispiel 3. Zur Herstellung der zweiten Monomerlösung wurden 88 g 15-fach ethoxiliertes Trimethylolpropantriacrylat eingesetzt.
Die wasserabsorbierenden Polymerpartikel hatten folgende Eigenschaften:

| | |
|---|---|
| CRC | 34,9 g/g |
| AUL0.7psi | 27,9 g/g |
| Extrahierbare | 3,5 Gew.-% |
| SFC | $50x10^{-7}$ $cm^3s/g$ |

Das mittlere E-Modul der äußeren Partikeloberfläche betrug 140 kPa, das mittlere E-Modul der Innenwand des Hohlraumes betrug 50 kPa und der Quotient der mittleren E-Module betrug 2,8.
Das mittlere E-Modul ist der Mittelwert aus 20 Einzelmessungen. Insgesamt 4 Einzelmessungen des E-Moduls der äußeren Partikeloberfläche ergaben einen Wert von weniger als 84 kPa.
Der mittlere Partikeldurchmesser betrug 370 μm.

Beispiel 5

Es wurde verfahren wie unter Beispiel 3. Zur Herstellung der zweiten Monomerlösung wurden 176 g 15-fach ethoxiliertes Trimethylolpropantriacrylat eingesetzt.
Die wasserabsorbierenden Polymerpartikel hatten folgende Eigenschaften:

| | |
|---|---|
| CRC | 34,0 g/g |
| AUL0.7psi | 29,3 g/g |
| Extrahierbare | 3,0 Gew.-% |
| SFC | 90 x $10^{-7}$ $cm^3s/g$ |

Das mittlere E-Modul der äußeren Partikeloberfläche betrug 180 kPa, das mittlere E-Modul der Innenwand des Hohlraumes betrug 60 kPa und der Quotient der mittleren E-Module betrug 3.
Das mittlere E-Modul ist der Mittelwert aus 20 Einzelmessungen. Insgesamt 5 Einzelmessungen des E-Moduls der äußeren Partikeloberfläche ergaben einen Wert von weniger als 108 kPa.
Der mittlere Partikeldurchmesser betrug 380 μm.

Beispiel 6 (Vergleichsbeispiel)

Es wurde verfahren wie in Beispiel 6 von WO 2006/077054 A1.
Die wasserabsorbierenden Polymerpartikel hatten folgende Eigenschaften:

| | |
|---|---|
| CRC | 21,2 g/g |
| AUL0.3psi | 17,6 g/g |
| Extrahierbare | 17,5 Gew.-% |
| SFC | 12 x $10^{-7}$ $cm^3s/g$ |

Das mittlere E-Modul der äußeren Partikeloberfläche betrug 80 kPa, das mittlere E-Modul der Innenwand des Hohlraumes betrug 40 kPa und der Quotient der mittleren E-Module betrug 2.
Das mittlere E-Modul ist der Mittelwert aus 20 Einzelmessungen. Insgesamt 4 Einzelmessungen des E-Moduls der äußeren Partikeloberfläche ergaben einen Wert von weniger als 48 kPa.
Der mittlere Partikeldurchmesser betrug 210 μm.

Beispiel 7 (Vergleichsbeispiel)

14,275 kg Natriumacrylat (37,5 gew.-%ige Lösung in Wasser) und 1,367 kg Acrylsäure wurden mit 0,358 kg Wasser, 22 g 15-fach ethoxiliertem Trimethylolpropantriacrylat und 80 g EDTA (10 gew.-%ige Lösung des Natriumsalzes von Ethylendiamintetraessigsäure in Wasser) gemischt. Die Lösung wurde nach Zugabe von 33 g 2,2'-Azobis[2-(2-imidazolin-2-yl)propan]dihydrochlorid (3 gew.-%ige Lösung in Wasser) und 110 g Wasserstoffperoxid (3 gew.-%ige Lösung in Wasser) in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen à 170 μm auf. Der Durchmesser der Vertropferplatte betrug 65 mm. Der Initiator wurde kurz vor dem Vertropfer über einen statischen Mischer mit der Monomerlösung gemischt.
Die wasserabsorbierenden Polymerpartikel hatten folgende Eigenschaften:

| | |
|---|---|
| CRC | 33,0 g/g |
| AUL0.7psi | 25,0 g/g |
| Extrahierbare | 7,0 Gew.-% |
| SFC | 10 x $10^{-7}$ $cm^3s/g$ |

Das mittlere E-Modul der äußeren Partikeloberfläche betrug 90 kPa, das mittlere E-Modul der Innenwand des Hohlraumes betrug 40 kPa und der Quotient der mittleren E-Module betrug 2,25.

Das mittlere E-Modul ist der Mittelwert aus 20 Einzelmessungen. Insgesamt 3 Einzelmessungen des E-Moduls der äußeren Partikeloberfläche ergaben einen Wert von weniger als 54 kPa.

Der mittlere Partikeldurchmesser betrug 360 μm.

**Patentansprüche**

1. Verfahren zur Herstellung wasserabsorbierender Polymerpartikel durch Polymerisation von Tropfen, enthaltend

   a) mindestens ein ethylenisch ungesättigtes Monomer,
   b) mindestens eines Vernetzers,
   c) mindestens einen Initiator,
   d) Wasser,
   in einer die Tropfen umgebenden Gasphase, wobei die Tropfen erzeugt werden, indem eine erste Monomerlösung mit einer zweiten Monomerlösung umhüllt wird, **dadurch gekennzeichnet, dass** die zweite Monomerlösung zu einem höher vernetzten Polymer polymerisiert als die erste Monomerlösung.

2. Verfahren gemäß Anspruch 1, **dadurch gekennzeichnet, dass** die molare Vernetzerkonzentration in der zweiten Monomerlösung mindestens 10% höher ist als in der ersten Monomerlösung.

3. Verfahren gemäß Anspruch 1 oder 2, **dadurch gekennzeichnet, dass** die erste Monomerlösung über mindestens eine Bohrung in die Gasphase dosiert wird und der Druckverlust über die Bohrung weniger als 2,5 bar beträgt.

4. Verfahren gemäß einem der Ansprüche 1 bis 3, **dadurch gekennzeichnet, dass** die zweite Monomerlösung über einen die Zuführung der ersten Monomerlösung umhüllenden Ringspalt dosiert wird.

5. Verfahren gemäß einem der Ansprüche 1 bis 4, **dadurch gekennzeichnet, dass** der Ringspalt eine Breite von mindestens 50 μm aufweist.

6. Verfahren gemäß einem der Ansprüche 1 bis 5, **dadurch gekennzeichnet, dass** die Tropfen einen mittleren Durchmesser von mindestens 200 μm aufweisen.

7. Verfahren gemäß einem der Ansprüche 1 bis 6, **dadurch gekennzeichnet, dass** das Monomer a) mindestens eine Säuregruppe aufweist.

8. Verfahren gemäß Anspruch 7, **dadurch gekennzeichnet, dass** die Säuregruppen des Monomeren a) zumindest teilweise neutralisiert sind.

9. Verfahren gemäß einem der Ansprüche 1 bis 8, **dadurch gekennzeichnet, dass** Monomer a) zu mindestens 50 mol-% Acrylsäure ist.

10. Verfahren gemäß einem der Ansprüche 1 bis 9, **dadurch gekennzeichnet, dass** die erhaltenen Polymerpartikel in mindestens einem weiteren Verfahrensschritt getrocknet und/oder nachvemetzt werden.

11. Wasserabsorbierende Polymerpartikel, wobei die Partikel eine Zentrifugenretentionskapazität von mindestens 30 g/g (EDANA Testmethode Nr.441.2-02), eine Permeabilität von mindestens 30 x $10^{-7}$ $cm^3s/g$ (bestimmt durch die im Absatz "Flüssigkeitsweiterleitung" der Beschreibung offenbarte Methode) und weniger als 30% der gemessenen E-Module der äußeren Partikeloberfläche einen Wert von weniger als 60% des mittleren E-Modules aufweisen (bestimmt durch die im Absatz "mittleres E-Modul" der Beschreibung offenbarte Methode).

12. Polymerpartikel gemäß Anspruch 11, **dadurch gekennzeichnet, dass** die Polymerpartikel eine Absorption unter einem Druck von 4,83 kPa (AUL0.7psi) von mindestens 20 g/g aufweisen (EDANA Testmethode Nr. 442.2-02).

13. Polymerpartikel gemäß Anspruch 11 oder 12, **dadurch gekennzeichnet, dass** die äußere Partikeloberfläche der Polymerpartikel ein mittleres E-Modul von mindestens 100 kPa aufweist (EDANA Testmethode Nr.470.2-02).

**14.** Polymerpartikel gemäß einem der Ansprüche 11 bis 13, **dadurch gekennzeichnet, dass** die Polymerpartikel weniger als 10 Gew.-% extrahierbare Anteile enthalten.

**15.** Polymerpartikel gemäß einem der Ansprüche 11 bis 14, **dadurch gekennzeichnet, dass** die Polymerpartikel mindestens einen Hohlraum im Partikelinneren enthalten.

**16.** Polymerpartikel gemäß Anspruch 15, **dadurch gekennzeichnet, dass** das Verhältnis von maximalem Durchmesser des Hohlraumes zu maximalem Durchmesser des Polymerpartikels mindestens 0,1 beträgt.

**17.** Polymerpartikel gemäß Anspruch 15 oder 16, **dadurch gekennzeichnet, dass** der Quotient aus mittlerem E-Modul der äußeren Partikeloberfläche und mittlerem E-Modul der Innenwand des Hohlraumes mindestens 2,5 beträgt.

**18.** Polymerpartikel gemäß einem der Ansprüche 11 bis 17, **dadurch gekennzeichnet, dass** die Polymerpartikel zu mindestens 50 mol-% zumindest teilweise neutralisierte polymerisierte Acrylsäure enthalten.

**19.** Polymerpartikel gemäß einem der Ansprüche 11 bis 18, **dadurch gekennzeichnet, dass** die Polymerpartikel einen mittleren Durchmesser von mindestens 200 μm aufweisen.

**20.** Verwendung der Polymerpartikel gemäß einem der Ansprüche 11 bis 19 zur Herstellung von Hygieneartikeln.

**21.** Hygieneartikel, enthaltend Polymerpartikel gemäß einem der Ansprüche 11 bis 19.

**Claims**

**1.** A process for preparing water-absorbing polymer beads by polymerizing droplets comprising

a) at least one ethylenically unsaturated monomer,
b) at least one crosslinker,
c) at least one initiator,
d) water,
in a gas phase surrounding the droplets, the droplets being obtained by enveloping a first monomer solution with a second monomer solution, wherein the second monomer solution polymerizes to give a more highly crosslinked polymer than the first monomer solution.

**2.** The process according to claim 1, wherein the molar crosslinker concentration in the second monomer solution is at least 10% higher than in the first monomer solution.

**3.** The process according to claim 1 or 2, wherein the first monomer solution is metered into the gas phase through at least one bore and the pressure drop through the bore is less than 2.5 bar.

**4.** The process according to any of claims 1 to 3, wherein the second monomer solution is metered in through an annular gap which encloses the feed of the first monomer solution.

**5.** The process according to any of claims 1 to 4, wherein the annular gap has a width of at least 50 μm.

**6.** The process according to any of claims 1 to 5, wherein the droplets have a mean diameter of at least 200 μm.

**7.** The process according to any of claims 1 to 6, wherein the monomer a) has at least one acid group.

**8.** The process according to claim 7, wherein the acid groups of the monomer a) have been at least partly neutralized.

**9.** The process according to any of claims 1 to 8, wherein the monomer a) is acrylic acid to an extent of at least 50 mol%.

**10.** The process according to any of claims 1 to 9, wherein the resulting polymer beads are dried and/or postcrosslinked in at least one further process step.

**11.** Water-absorbing polymer beads which have a centrifuge retention capacity of at least 30 g/g (EDANA test method

No. 441.2-02) and a permeability of at least 30 x $10^{-7}$ $cm^3s/g$ (determined by the method disclosed in the "Saline flow conductivity" paragraph in the description), and less than 30% of the measured moduli of elasticity of the outer bead surface have a value of less than 60% of the mean modulus of elasticity (determined by the method disclosed in the "Mean modulus of elasticity" paragraph in the description).

**12.** The polymer beads according to claim 11, which have an absorbency under a load of 4.83 kPa (AUL0.7psi) of at least 20 g/g (EDANA test method No. 442.2-02).

**13.** The polymer beads according to claim 11 or 12, wherein the outer bead surface of the polymer beads has a mean modulus of elasticity of at least 100 kPa (EDANA test method No. 470.2-02).

**14.** The polymer beads according to any of claims 11 to 13, which comprise less than 10% by weight of extractables.

**15.** The polymer beads according to any of claims 11 to 14, which comprise at least one cavity in the bead interior.

**16.** The polymer beads according to claim 15, wherein the ratio of maximum diameter of the cavity to maximum diameter of the polymer bead is at least 0.1.

**17.** The polymer beads according to claim 15 or 16, wherein the quotient of mean modulus of elasticity of the outer bead surface and mean modulus of elasticity of the inner wall of the cavity is at least 2.5.

**18.** The polymer beads according to any of claims 11 to 17, which comprise at least partly neutralized polymerized acrylic acid to an extent of at least 50 mol%.

**19.** The polymer beads according to any of claims 11 to 18, which have a mean diameter of at least 200 μm.

**20.** The use of the polymer beads according to any of claims 11 to 19 for producing hygiene articles.

**21.** A hygiene article comprising polymer beads according to any of claims 11 to 19.

**Revendications**

**1.** Procédé pour la préparation de particules polymères absorbant l'eau par polymérisation de gouttes, contenant

a) au moins un monomère éthyléniquement insaturé,
b) au moins un réticulant,
c) au moins un initiateur,
d) de l'eau,
dans une phase gazeuse entourant les gouttes, les gouttes étant produites en ce qu'une première solution de monomères est entourée par une deuxième solution de monomères, **caractérisé en ce que** la deuxième solution de monomères polymérise en un polymère plus fortement réticulé que la première solution de monomères.

**2.** Procédé selon la revendication 1, **caractérisé en ce que** la concentration molaire en réticulant dans la deuxième solution de monomères est supérieure d'au moins 10% à celle dans la première solution de monomères.

**3.** Procédé selon la revendication 1 ou 2, **caractérisé en ce que** la première solution de monomères est dosée dans la phase gazeuse via au moins un trou et la perte de charge via le trou est inférieure à 2,5 bars.

**4.** Procédé selon l'une quelconque des revendications 1 à 3, **caractérisé en ce que** la deuxième solution de monomères est dosée via une fente annulaire entourant l'alimentation de la première solution de monomères.

**5.** Procédé selon l'une quelconque des revendications 1 à 4, **caractérisé en ce que** la fente annulaire présente une largeur d'au moins 50 μm.

**6.** Procédé selon l'une quelconque des revendications 1 à 5, **caractérisé en ce que** les gouttes présentent un diamètre moyen d'au moins 200 μm.

**7.** Procédé selon l'une quelconque des revendications 1 à 6, **caractérisé en ce que** le monomère a) présente au moins un groupe acide.

**8.** Procédé selon la revendication 7, **caractérisé en ce que** les groupes acides du monomère a) sont au moins partiellement neutralisés.

**9.** Procédé selon l'une quelconque des revendications 1 à 8, **caractérisé en ce que** le monomère a) est, à raison d'au moins 50% en mole, de l'acide acrylique.

**10.** Procédé selon l'une quelconque des revendications 1 à 9, **caractérisé en ce que** les particules polymères obtenues sont séchées et/ou post-réticulées dans au moins une autre étape de procédé.

**11.** Particules polymères absorbant l'eau, où les particules présentent une capacité de rétention à la centrifugeuse d'au moins 30 g/g (méthode de test EDANA n° 441.2-02), une perméabilité d'au moins 30 x $10^{-7}$ $cm^3$ s/g (déterminée par la méthode divulguée dans le paragraphe "Conductivité de flux salin" de la description) et moins de 30% du module E mesuré de la surface extérieure des particules présentent une valeur de moins de 60% du module E moyen (déterminé par la méthode divulguée dans la paragraphe "module E moyen" de la description).

**12.** Particules polymères selon la revendication 11, **caractérisées en ce que** les particules polymères présentent une adsorption à une pression de 4,83 kPa (AUL0.7 psi) d'au moins 20 g/g (méthode de test EDANA n° 442.2-02).

**13.** Particules polymères selon la revendication 11 ou 12, **caractérisées en ce que** la surface extérieure des particules polymères présente un module E moyen d'au moins 100 kPa (méthode de test EDANA n° 470.2-02).

**14.** Particules polymères selon l'une quelconque des revendications 11 à 13, **caractérisées en ce que** les particules polymères contiennent moins de 10% en poids de proportions pouvant être extraites.

**15.** Particules polymères selon l'une quelconque des revendications 11 à 14, **caractérisées en ce que** les particules polymères contiennent au moins un espace creux à l'intérieur des particules.

**16.** Particules polymères selon la revendication 15, **caractérisées en ce que** le rapport du diamètre maximal de l'espace creux au diamètre maximal de la particule polymère est d'au moins 0,1.

**17.** Particules polymères selon la revendication 15 ou 16, **caractérisées en ce que** le quotient du module E moyen de la surface extérieure des particules et du module E moyen de la paroi interne de l'espace creux est d'au moins 2,5.

**18.** Particules polymères selon l'une quelconque des revendications 11 à 17, **caractérisées en ce que** les particules polymères contiennent à raison d'au moins 50% en mole de l'acide acrylique polymérisé au moins partiellement neutralisé.

**19.** Particules polymères selon l'une quelconque des revendications 11 à 18, **caractérisées en ce que** les particules polymères présentent un diamètre moyen d'au moins 200 µm.

**20.** Utilisation des particules polymères selon l'une quelconque des revendications 11 à 19 pour la production d'articles hygiéniques.

**21.** Articles hygiéniques contenant des particules polymères selon l'une quelconque des revendications 11 à 19.

Figur 1

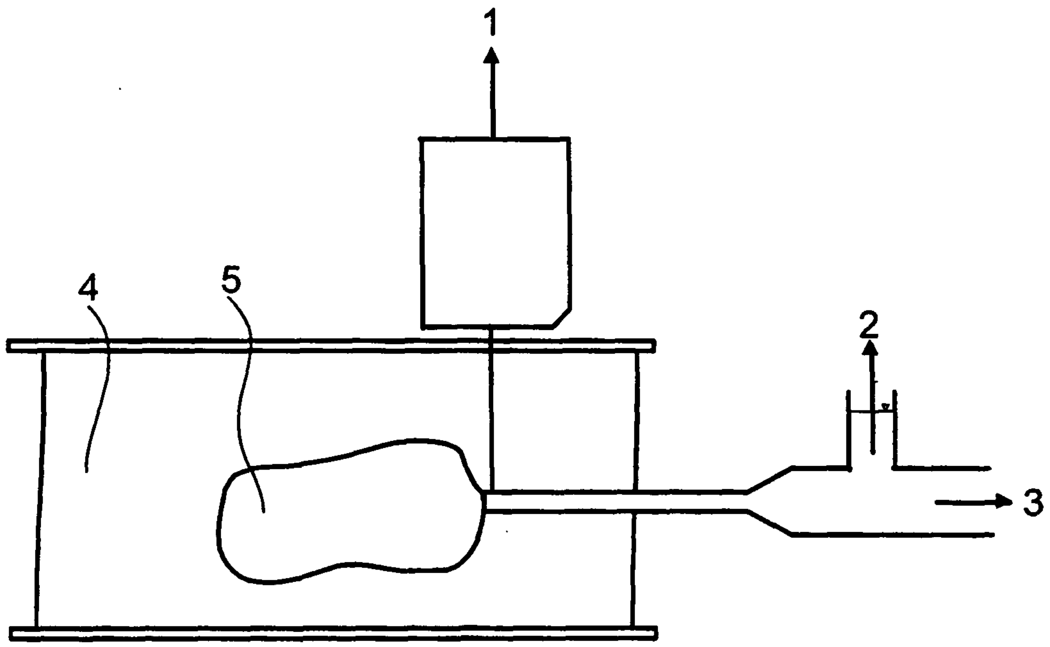

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 348180 A1
- JP 5132503 A
- WO 9640427 A1
- US 5269980 A
- DE 10314466 A1
- DE 10340253 A1
- DE 102004024437 A1
- WO 2006077054 A1
- DE 102006001596
- EP 2006062252 W
- DE 102004042946 A1
- DE 102004042948 A1
- DE 102004042955 A1
- DE 102005019398
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 343427 A2
- DE 4308842 A1
- EP 83022 A2
- EP 543303 A1
- EP 937736 A2
- DE 3314019 A1
- DE 3523617 A1
- EP 450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 200331482 A1
- EP 640330 A1


### In der Beschreibung aufgeführte Nicht-Patentliteratur


- **F.L. Buchholz ; A.T. Graham.** Modern Superabsorbent Polymer Technology. Wiley-VCH, 1998, 71-103